(19) **Europäisches Patentamt**
**European Patent Office**
**Office européen des brevets**

(11) **EP 3 937 758 B1**

(12) **EUROPEAN PATENT SPECIFICATION**

(45) Date of publication and mention
of the grant of the patent:
**11.03.2026 Bulletin 2026/11**

(21) Application number: **20717467.3**

(22) Date of filing: **12.03.2020**

(51) International Patent Classification (IPC):
**A61B 5/00** (2006.01) **G01B 9/02** (2022.01)

(52) Cooperative Patent Classification (CPC):
**A61B 5/0066; A61B 5/0088; A61B 5/7207;**
**A61B 5/7221; G01B 9/02004; G01B 9/02019;**
**G01B 9/02027; G01B 9/02076; G01B 9/02091;**
A61B 2560/0233; A61B 2562/043; A61B 2562/046;
G01B 9/02069

(86) International application number:
**PCT/US2020/022362**

(87) International publication number:
**WO 2020/186041 (17.09.2020 Gazette 2020/38)**

(54) **HIGH-SPEED, DENTAL OPTICAL COHERENCE TOMOGRAPHY SYSTEM**

OPTISCHES DENTALES KOHÄRENZTOMOGRAFIESYSTEM MIT HOHER GESCHWINDIGKEIT

SYSTÈME DE TOMOGRAPHIE PAR COHÉRENCE OPTIQUE DENTAIRE À GRANDE VITESSE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.03.2019 US 201962817195 P**

(43) Date of publication of application:
**19.01.2022 Bulletin 2022/03**

(73) Proprietor: **Dental Imaging Technologies**
**Corporation**
**Quakertown PA 18951 (US)**

(72) Inventors:
• **TAO, Xiaodong**
**Rochester, NY 14445 (US)**
• **WONG, Victor, C.**
**Rochester, NY 14445 (US)**
• **FAN, Chuanhmao**
**Rochester, NY 14623 (US)**

(74) Representative: **Hoffmann Eitle**
**Patent- und Rechtsanwälte PartmbB**
**Arabellastraße 30**
**81925 München (DE)**

(56) References cited:
**US-A1- 2011 109 911     US-A1- 2017 363 415**

• **BROOKE KRAJANCICH ET AL: "Handheld**
**optical palpation of turbid tissue with motion-**
**artifact correction", BIOMEDICAL OPTICS**
**EXPRESS, vol. 10, no. 1, 18 December 2018**
**(2018-12-18), United States, pages 226,**
**XP055704026, ISSN: 2156-7085, DOI: 10.1364/**
**BOE.10.000226**
• **HUI WANG ET AL: "Extending the effective**
**imaging range of Fourier-domain optical**
**coherence tomography using a fiber optic**
**switch", OPTICS LETTERS, OPTICAL SOCIETY**
**OF AMERICA, US, vol. 33, no. 22, 15 November**
**2008 (2008-11-15), pages 2632 - 2634,**
**XP001520926, ISSN: 0146-9592, DOI: 10.1364/**
**OL.33.002632**
• **ANDREAS WARTAK ET AL: "Adaptable**
**switching schemes for time-encoded**
**multichannel optical coherence tomography",**
**JOURNAL OF BIOMEDICAL OPTICS, vol. 23, no.**
**05, 24 May 2018 (2018-05-24), pages 1,**
**XP055704145, ISSN: 1083-3668, DOI: 10.1117/**
**1.JBO.23.5.056010**

• MANSIK JEON ET AL: "Full-range k-domain linearization in spectral-domain optical coherence tomography", APPLIED OPTICS, OPTICAL SOCIETY OF AMERICA, WASHINGTON, DC, US, vol. 50, no. 8, 10 March 2011 (2011-03-10), pages 1158 - 1163, XP001561050, ISSN: 0003-6935, [retrieved on 20110308], DOI: 10.1364/AO.50.001158

• YIH MIIN LIEW ET AL: "Motion correction of in vivo three-dimensional optical coherence tomography of human skin using a fiducial marker", BIOMEDICAL OPTICS EXPRESS, vol. 3, no. 8, 1 August 2012 (2012-08-01), United States, pages 1774, XP055518828, ISSN: 2156-7085, DOI: 10.1364/BOE.3.001774

## Description

## FIELD OF THE INVENTION

The present invention relates generally to dental and maxillofacial optical coherence tomography (OCT) imaging and, more particularly, to a handheld intraoral OCT apparatus with improved speed and increased imaging range and methods related to same.

## BACKGROUND

Optical coherence tomography (OCT) is a non-invasive imaging technique that employs interferometric principles to obtain high resolution, cross-sectional tomographic images that characterize the depth structure of a sample. Particularly suitable for *in vivo* imaging of human tissue, OCT has shown its usefulness in a range of biomedical research and medical imaging applications, such as in ophthalmology, dermatology, oncology, and other fields, as well as in ear-nose-throat (ENT) and dental imaging.

OCT has been described as a type of "optical ultrasound", imaging reflected energy from within living tissue to obtain cross-sectional data. In an OCT imaging system, light from a wide-bandwidth source, such as a super luminescent diode (SLD) or other light source, is directed along two different optical paths: a reference arm or path of known optical path length and a sample arm or path that illuminates the tissue or other subject under study. Reflected and back-scattered light from the reference and sample arms is then recombined in the OCT apparatus and interference effects are used to determine characteristics of the surface and near-surface underlying structure of the sample. Interference data can be acquired by rapidly scanning the illumination across the sample. At each of several thousand points along the sample surface, the OCT apparatus obtains an interference profile which can be used to reconstruct an A-scan with an axial depth into the material that is largely a factor of light source coherence. For most tissue imaging applications, OCT uses broadband illumination sources and can provide image content at depths of up to a few millimeters (mm).

Among challenges for a hand-held, dental and maxillofacial optical coherence tomography (OCT) scanning system are obtaining sufficient imaging speed and having suitable imaging range for use as a diagnostic aid. While high speed is a key factor in minimizing imaging artifacts resulting from the motion of a hand-held scanner, the high-speed raster scanning used in most OCT systems induces artifacts such wobble, skew, and spatial aliasing. Artifact correction based on postprocessing fails to provide reliable results and the postprocessing time is often too long for real time imaging. Obtaining sufficient imaging range enables the OCT imaging apparatus to show more effectively the condition of tissue or other material beneath the surface of the imaged tooth or other sample.

One way to increase image acquisition speed is to utilize a high speed, swept laser source and a high-speed scanner. Real time OCT imaging has been demonstrated by using a high-speed Fourier Domain Mode Locking (FDML) laser. However, the FDML laser's increased complexity and high cost limits its application in dental applications. Additionally, an OCT system using an FDML laser can only provide a limited imaging range.

Recent availability of micro-electromechanical system (MEMS)-based swept sources, such as tunable vertical cavity surface emitting lasers, capable of providing high sweep rate operation in the megahertz range, may help to achieve increases in scanning speed, allowing faster image acquisition. Unfortunately, however, use of high-sweep rate swept sources has some disadvantages. For example, expensive, high-speed digitizers are required to achieve an increased imaging range when using a high rate swept source OCT system. Additionally, image quality suffers significantly at high sampling rates, because of photon noise and electrical noise.

Improvements in OCT acquisition speed are needed to make OCT more usable, but must be accomplished without significantly increasing cost, without compromising image quality, and without limiting imaging range. There is a need for a high speed, dental OCT system that offers improvement in high-speed image acquisition and enhanced imaging range, but without relying on a very high sweep rate swept source.

Moreover, reference is made to US 2011 / 109 911 A1 disclosing an optical interferometer used to provide simultaneous measurements over multiple path lengths and employment methods combining scanning regimes and modes of operation in 3D imaging of moving tissue such as the eye, heart, or moving embryos or functional/low noise using angular compounding or polarisation.

## SUMMARY

In accordance with the present invention, a hand-held dental optical coherence tomography system and a method for hand-held dental optical coherence tomography for imaging a sample as set forth in claims 1 and 13, respectively, is provided. Further embodiments of the invention are inter alia disclosed in the dependent claims. Broadly described, the present invention comprises a high speed, dental OCT system, including apparatus and methods, that offers improvement in high-speed image acquisition and enhanced imaging range, but without relying on a very high sweep rate swept source. According to one aspect of the present invention, there is provided a dental optical coherence tomography system for scanning a sample that comprises (a) a swept source laser configured to generate an output light having a range of light wavelengths, (b) two or more optical channels that each include (i) a reference path and a sample path for the output light from the swept source laser and (ii) a corresponding detector that is configured to provide an output signal according to combined light from the sample and reference paths, the detector being operable to output a signal that characterizes back-reflected or back-scattered light returned from the sample path and over a range of depths below a sample surface, (c) a scanning reflector that is configured to simultaneously direct sample path output light from each of the two or more optical channels toward the sample surface and to direct the returned light from the sample to the corresponding sample path and detector, the scanner being incorporated into a handheld probe, and (d) a processor that is in signal communication with the detector for each optical channel and that is configured to record and store results from the output signals received from each detector.

The foregoing and other aspects, features, and advantages of the present invention will be apparent from the following more particular description of example embodiments thereof and the accompanying drawings.

## BRIEF DESCRIPTION OF THE DRAWINGS

FIG. 1 is a schematic diagram displaying a conventional swept-source OCT (SS-OCT) apparatus.

FIG. 2A displays a schematic representation of a scanning operation for obtaining a B-scan.

FIG. 2B displays an OCT scanning pattern for C-scan acquisition.

FIG. 3A is a schematic diagram that displays a high-speed intraoral OCT system having multiple channels according to an example embodiment of the present invention.

FIG. 3B is a schematic diagram displaying components that collimate, focus, and scan light from each channel.

FIG. 3C is a schematic diagram displaying a channel with an additional camera for viewing an imaged sample.

FIG. 4A displays a schematic for an apparatus using a one-dimensional array for providing output beams from multiple channels.

FIG. 4B is a schematic diagram displaying an apparatus using a two-dimensional array for providing output beams from multiple channels.

FIG. 5 is a schematic diagram displaying an apparatus for scanning multiple channels at different depths.

FIG. 6 is a schematic diagram displaying an apparatus for scanning multiple channels with different optical lengths for each sample arm.

FIG. 7 is a schematic diagram displaying use of a fiber array and optical switching for scanning a region of interest.

FIGs. 8A, 8B, and 8C display configurations for compensating depth shift between channels.

FIGs. 9A and 9B display how diffuse surface compensation helps to correct for mechanical drift in the reference arm.

FIG. 10 is a schematic diagram displaying an alternate embodiment of a swept-source OCT (SS-OCT) apparatus using polarization.

FIG. 11 is a schematic diagram showing a sequence for artifact removal using a reference feature.

## DETAILED DESCRIPTION OF EXAMPLE EMBODIMENTS

The following is a detailed description of example embodiments of the present invention with reference being made to the drawings in which the same reference numerals identify the same elements of structure or steps of a method in each of the several figures.

Where they are used in the context of the present disclosure, the terms "first", "second", and so on, do not necessarily denote any ordinal, sequential, or priority relation, but are simply used to more clearly distinguish one step, element, or set of elements from another, unless specified otherwise.

The general term "scanner" relates to an optical system that is energizable to project a scanned light beam of light, such as broadband near-IR (BNIR) light that is directed to the tooth surface through a sample arm and acquired, as reflected and scattered light returned in the sample arm, for measuring interference with light from a reference arm used in OCT imaging of a surface. The term "raster scanner" relates to the combination of hardware components that sequentially scan light toward uniformly spaced locations along a sample, as described in more detail below.

In the context of the present disclosure, the phrase "imaging range" relates to the effective distance (generally considered in the z-axis or A-scan direction) over which OCT measurement is available. The OCT beam is considered to be within focus over the imaging range. Image depth relates to imaging range, but has additional factors related to signal penetration through the sample tooth or other tissue.

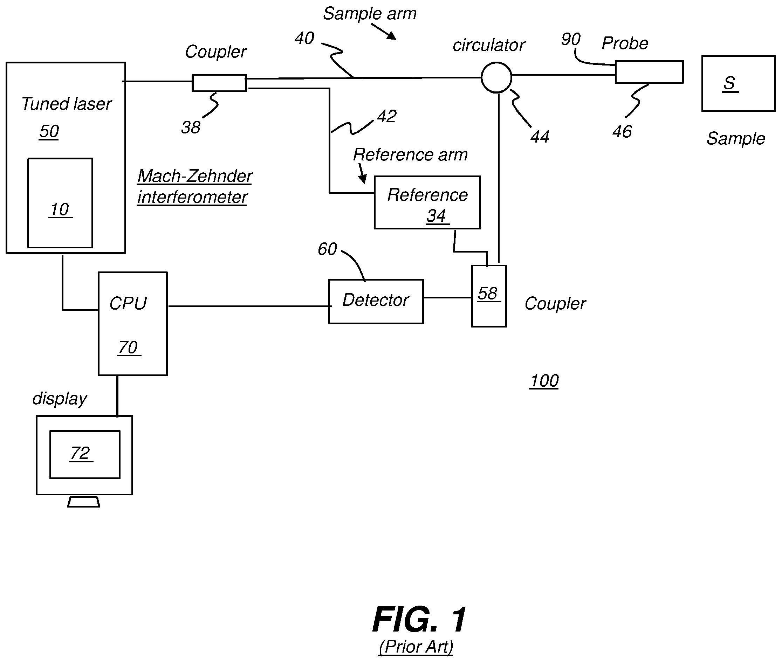

By way of example, the simplified schematic diagram of FIG. 1 displays the components of one type of OCT apparatus, here, a conventional swept-source OCT (SS-OCT) apparatus 100 using a Mach-Zehnder interferometer (MZI) system with a light source provided by a programmable filter 10 that is part of a tuned laser 50. For intraoral OCT, for example, laser 50 can be tunable over a range of frequencies (expressed in terms of wave-numbers k) corresponding to wavelengths between about 400 and 1600 nm. According to an embodiment of the present disclosure, a tunable range of about 60 nm bandwidth centered about 1300 nm is used for intraoral OCT.

In the FIG. 1 device, the variable tuned laser 50 output goes through a coupler 38 and to a sample arm 40 and a reference arm 42. The sample arm 40 signal goes through a circulator 44 and is directed for imaging of a sample S from a handpiece or probe 46. The sampled signal is directed back through circulator 44 and to a detector 60 through a coupler 58. The reference arm 42 signal is directed by a reference 34, which can be a mirror or a light guide, through coupler 58 to detector 60. The detector 60 may use a pair of balanced photodetectors configured to cancel common mode noise.

Control logic processor 70 (also sometimes referred to herein as "control processing unit CPU 70" or "CPU 70") is in signal communication with tuned laser 50 and its programmable filter 10 and with detector 60. Processor 70 can control the scanning function of probe 46 and store any needed calibration data for obtaining a linear response to scan signals. Processor 70 obtains and processes the output from detector 60. CPU 70 is also in signal communication with a display 72 for command entry and OCT results display.

It should be noted that the swept-source architecture of FIG. 1 is one example only; there are a number of ways in which the interferometer components could be arranged for providing swept-source OCT imaging.

Among the proposed strategies for obtaining higher image acquisition speeds in an OCT system is simply using a high sweep-rate swept source. However, as previously described above, the problem is more complex. Attempts to operate at faster sweep rates have led to increased cost and can yield disappointing results with regards to the diagnostic benefits and overall quality of the OCT image content.

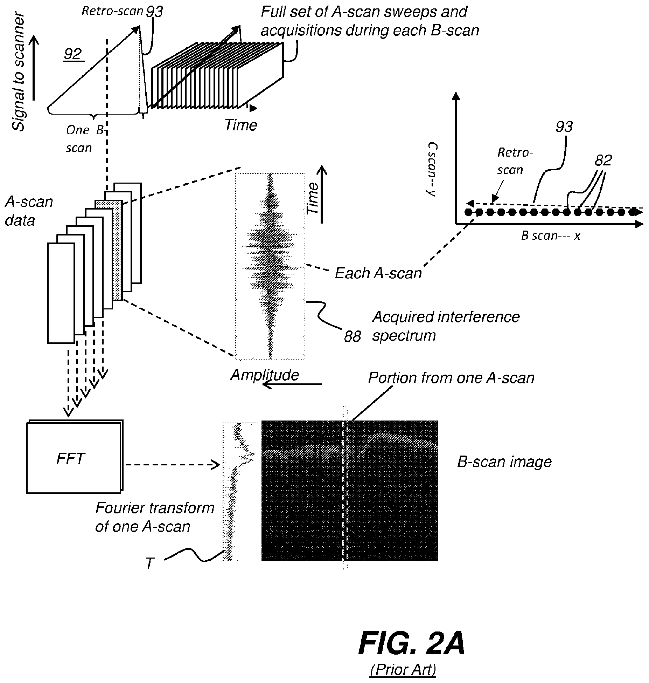

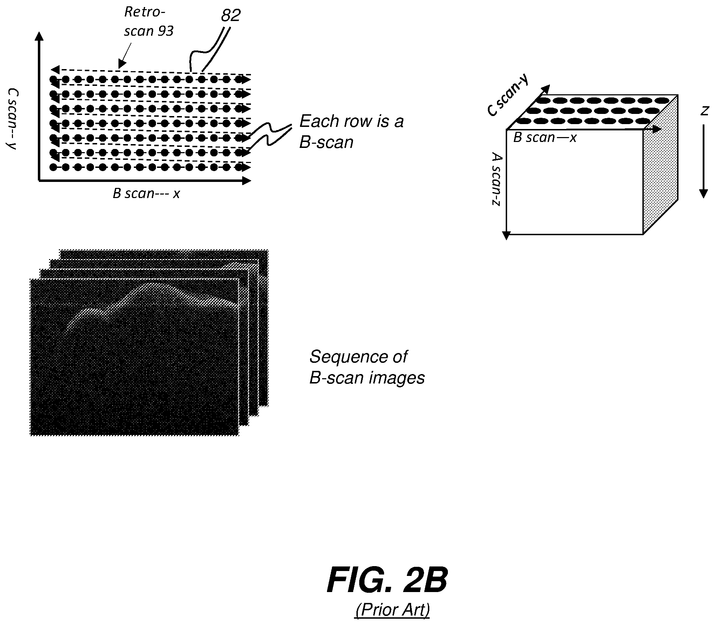

By way of further background, FIGs. 2A and 2B give an overview of the OCT scanning pattern as executed by probe 46. At each point in the scanning sequence, the OCT device performs an A-scan. A linear succession of A-scans then forms a B-scan. Successive B-scan rows, side-by-side, then form a C-scan which provides the three-dimensional ("3D") OCT image content for the sample "S".

FIG. 2A schematically displays the information acquired during each A-scan. The scan signal for obtaining each B-scan image has two linear sections in the example shown, with a scan portion 92, during which the scanning mirror is driven to direct the sampling beam from a beginning to an ending position, and a retro-scan 93, during which the scanning mirror is restored to its beginning position. An interference signal 88, shown with DC signal content removed, is acquired over the time interval for each point 82, wherein the signal is a function of the time interval required for the sweep, with the signal that is acquired indicative of the spectral interference fringes generated by combining the light from reference and feedback sample arms of the interferometer (FIG. 1). The Fast Fourier transform ("FFT") generates a transform "T" for each A-scan. One transform signal corresponding to an A-scan is shown by way of example in FIG. 2A.

From the above description, it can be appreciated that a significant amount of data is acquired during a single B-scan sequence. In order to process this data efficiently, a Fast Fourier Transform (FFT) is used, transforming the time-based signal data to corresponding frequency-based data from which image content can more readily be generated.

In Fourier domain OCT, the A scan corresponds to one line of spectrum acquisition which generates a line of depth (z-axis) resolved OCT signal. The B scan data generates a two-dimensional ("2-D") OCT image along the corresponding scanned line.

Raster scanning is used to obtain multiple B-scan data by incrementing the raster scanner 90 acquisition in the C-scan (y-axis) direction. This is represented schematically in FIG. 2B, which shows how 3-D volume information is generated using the A-, B-, and C-scan data.

The wavelength or frequency sweep sequence that is used at each A-scan point 82 can be modified from the ascending or descending wavelength sequence that is typically used. Arbitrary wavelength sequencing can alternatively be used. In the case of arbitrary wavelength sequencing, which may be useful for some particular implementations of OCT, only a portion of the available wavelengths are provided as a result of each sweep. In arbitrary wavelength sequencing, each wavelength can be randomly selected, in arbitrary sequential order, to be used in the OCT system during a single sweep. A-scan points 82 can be uniformly spaced from each other with respect to the x axis, providing a substantially equal x-axis distance between adjacent points 82 along any B-scan image. Similarly, the distance between lines of scan points 82 for each B scan can be uniform with respect to the y axis. X-axis spacing may differ from y-axis spacing; alternatively, spacing along these orthogonal axes of the scanned surface may be equal.

For conventional OCT approaches, image acquisition speed is related to factors of sweep rate and digitizer capability. Faster sweep rates can, in turn, allow improved A-scan frequencies, but at the cost of higher noise. High-speed digitization components are also needed at higher acquisition rates, with significant increase in component cost for the needed performance. Thus, there are some practical limits to scanning speed and overall OCT performance that can limit the use of OCT for chairside diagnosis and treatment.

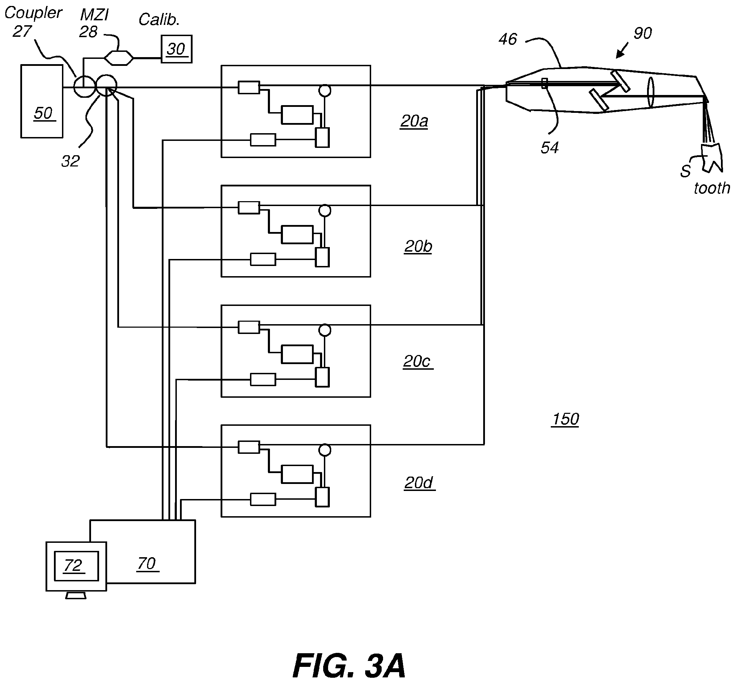

An example embodiment of the present disclosure, displayed schematically in FIG. 3A, addresses problems of image acquisition speed and the need for increased imaging range by using a multi-channel approach to dental OCT scanning and data acquisition. Referring to the schematic diagram of FIG. 3A, there is shown an exemplary high-speed intraoral OCT system 150 of the present disclosure having multiple channels. For increasing amounts of scanning speed, the number of channels "N" can be two, three, or four, such as the four channels 20a, 20b, 20c, and 20d shown in FIG. 3A. Additionally, five or more channels could be used, following the overall pattern described for four channels herein.

The scanner 90 within probe 46 directs light originating from swept-wavelength laser source 50 in multiple channels to the tooth or other sample "S".

As illustrated in FIG. 3A, a fiber coupler 27 splits off a small portion of the laser light to an MZI 28. The interference light from the MZI is collected by a photodetector and additional circuit 30 to provide K-clock (K-trigger) signals, which are timing control triggers having equal wavenumber spacing defined in time. Given equal spacing of these signals, the OCT signal sampled with the K-clock timing is linear in wavenumber space. Alternately, the OCT signal can be resampled into a linear wavenumber space using the interference signal from MZI 28 (zero crossing of the Mach-Zehnder interference (MZI) signal can be used to generate K-trigger signals to prompt the acquisition of SS-OCT signals). The bulk of the swept-source laser 50 light output is fed into the multi-channel system for OCT imaging through a splitter 32, such as a PLC (Planar Lightwave Circuit) splitter. In each channel, the light illuminates a fiber optic interferometer that has a circulator 44 and a 90/10 fiber coupler 38 that splits light into reference and sample arms 42, 40 (FIG. 1). The system can optionally include additional detectors and optical components to provide polarization sensitive optical coherence tomography.

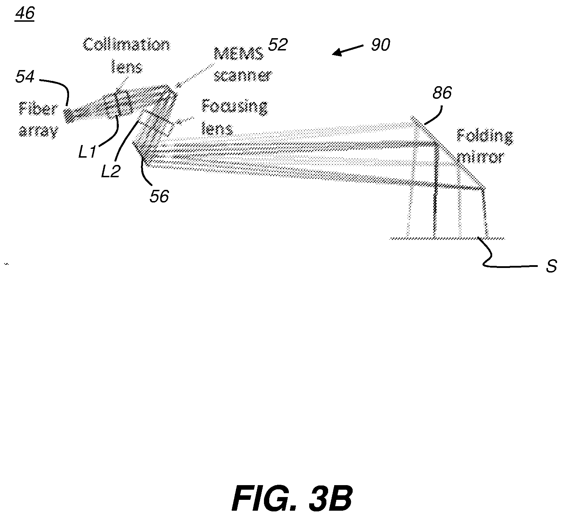

FIG. 3B displays probe 46 components that collimate, focus, and scan light from each of the four channels 20a, 20b, 20c, and 20d. As displayed in the schematic of FIG. 3B, the multi-channel sampling arms are connected with a fiber array 54 inside of the scanner handpiece, probe 46, which can be used intraorally or extraorally. Connection of the variable wavelength light is via a ribbon fiber (not shown). The fiber array 54 aligns the optical fiber cores precisely with desired pitch. The light from the fiber array goes through a collimation lens L1 and to a micro-electromechanical systems (MEMS) scanner 52. Scanned light then goes through a focusing lens L2 as shown in FIG. 3B. This focused light reflects from a first folding mirror surface 56 and a second folding mirror surface 86 and is directed to sample S. Multiple spots are focused on the sample S surface with desired spacing; each spot is from one of the multiple channels 20a, 20b, 20c, and 20d.

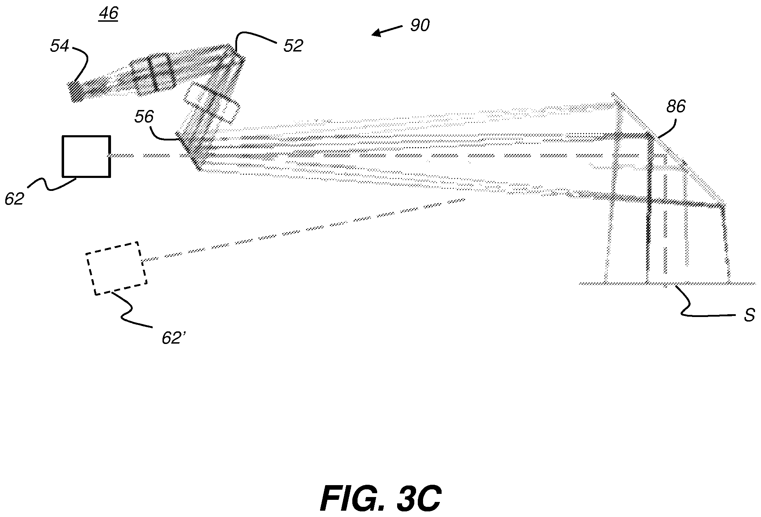

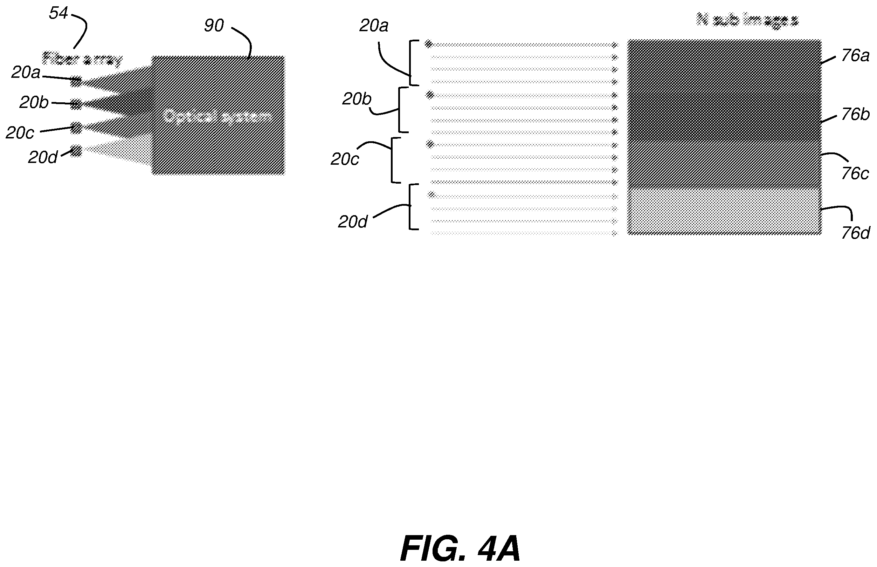

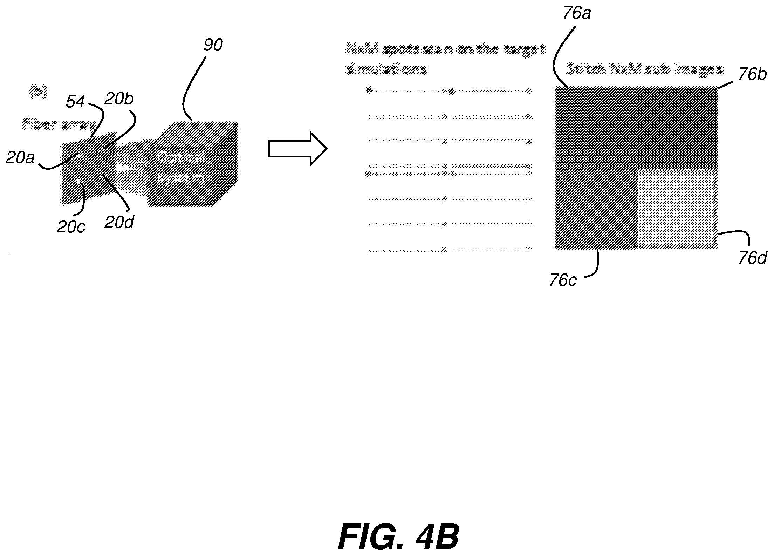

As is displayed in the schematic of FIG. 3C, probe 46 can optionally include other components such as, for example, a camera 62 for obtaining color information or to assist in probe movement. Where camera 62 is used, surface 56 can be a dichroic surface, treated to reflect the IR light used for OCT scanning and to transmit visible light to the camera 62. A camera can alternately be provided at an oblique angle with respect to optical axis OA; by way of example, an alternate position of a camera 62', which can be a second camera or the only camera, is displayed in FIG. 4.

Fiber array 54 within probe 46 can have a number of different configurations. FIG. 4A displays fiber array 54 arranged in line as a one-dimensional ("1D") array that simultaneously provides an output beam from each channel 20a, 20b, 20c, and 20d. The 1D array configuration can be used to direct the scanned beams to multiple spots, aligned on the target sample S. Scanning of a number N of illumination beams in this manner can be used to generate a number N of adjacent sub-images, shown as sub-images 76a, 76b, 76c, and 76d in the four-channel example of FIG. 4A. Processing software can then be used to stitch together the N adjacent images that lie along the scan line.

In scanning with a one-dimensional optical array using the FIG. 4A arrangement, the field of view (FOV) is divided in number of strips. Each focused spot from a channel scans only a small sub-region of the FOV. The reflected light from each focused spot at the sample is collected by probe 46 optics and is guided to the sampling arms of each channel. Light beams from the sample and reference arms 40 and 42 (FIG. 1) are recombined in the detection arms through a 50/50 coupler 58. Interference fringes that are formed are detected by balanced photo detectors or other mechanism in detector 60. The analogue signal from the balanced photo detector 60 can be digitized by a data acquisition card. The image volume from each channel can be generated using an OCT reconstruction algorithm. Finally, a reconstruction of the complete scanned image volume can be formed by stitching together the different sub-image volumes.

FIG. 4B displays an alternate arrangement using a 2x2 fiber array 54 to scan the FOV. This arrangement generates sub-image content as an array of images for stitching.

Since each channel scans only part of the field of view, the multi-channel system can achieve a much faster speed as compared to a single channel system. Using N multiple channels, scanning simultaneously, the complete FOV can be scanned in a fraction 1/N of the time required for the conventional single-channel arrangement.

Because the source laser output is split between N channels, some increase in laser power is needed in order to provide multi-channel OCT imaging capability. According to an embodiment of the present disclosure, a 40 mW laser is used to drive four channels, with output power subdivided to provide 10 mW in each channel.

In general, to achieve the same scanning speed, the swept laser source in an N-channel system only requires 1/N the sweep rate used in a single channel system. Lowering of the sweep rate accordingly lowers the digitization speed requirement of the data acquisition card, which can dramatically reduce the system cost.

To achieve the same imaging range, the frequency of the OCT signal, $f_{OCT}$, can be much lower with the multi-channel system than the frequency used in a single channel system. $f_{OCT}$ may be expressed as follows:

$$f_{OCT} = \frac{f_s \Delta \lambda Z}{\alpha \lambda^2},$$

wherein:

$\Delta\lambda$ is the bandwidth of the laser spectrum;
$\lambda$ is the central wavelength;
Z is the imaging range;
$\alpha$ is the duty cycle of the laser; and
$f_s$ is the frequency of the swept laser source.

Since, in an N-channel system, the frequency of the OCT signal is only 1/N of the frequency used in a single channel system, the digitizer can operate at a lower sampling rate. Thus, N-channel design can reduce both cost and system noise. Alternatively, if the same high-speed digitizer that is used for a single scanner OCT probe is used in an N-channel system, performance can be improved, at up to N times of the imaging range.

Variable Range Scanning

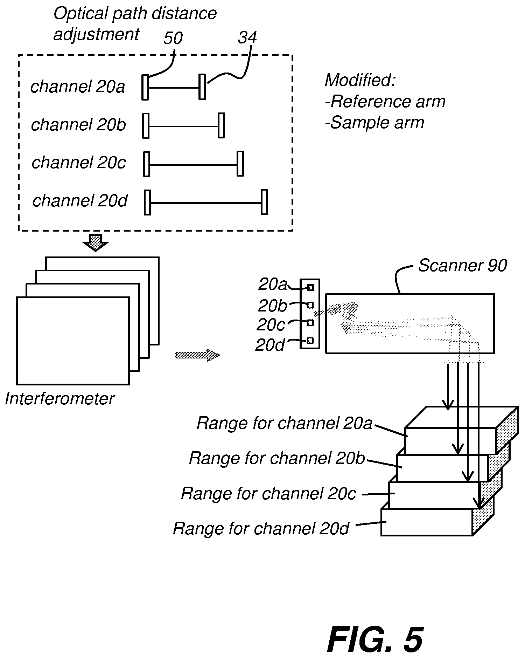

The multi-channel system also has the ability to extend the effective imaging range of the scanner without impact on the sampling rate. By introducing additional optical path difference (OPD) in the reference arm or the sampling arm, the beam from each channel can scan a different range of the target as shown schematically in FIG. 5. The range can be extended by factor of N, when an N channel system is used. However, this configuration may reduce the scanning speed over other arrangements, since each channel needs to scan the whole field of view.

By simultaneously scanning N channels and using image processing to stitch together the image content of the individual channels, embodiments of the present disclosure can process the corresponding image content in parallel and significantly reduce the overall scan time needed for OCT imaging over a given sample region and at desired scanning range.

Simultaneous multichannel scanning, with each channel scanning at a different range, effectively expands the overall imaging range available from the OCT scanner. The scanning arrangement of FIG. 5 shows schematically how variable range within a channel can be achieved within the interferometry subsystem for the channel, according to an example embodiment of the present invention. By varying the relative optical path lengths of reference and sample arms or paths 42 and 40, respectively, in each channel (FIG. 1), the scanned range in the z-direction for each individual channel can be modified.

Within the interferometry system for each channel, the reference arm 42 typically includes some type of mirror or other reflective surface. The distance that light travels towards and back from the reflective surface, that is, the optical path delay for the reference arm, directly relates to a particular range within the sampled material. Thus, by adjusting the optical distance between the reflective or back-scattering material and interferometry combining components, returned light from variable depths within the sample contributes to the detection signal. An alternate approach for scanning at different range, not shown in FIG. 5, changes the optical path delay of the sampling arm for each channel.

Methods for changing the optical path delay can include adding a length of optical fiber between two points along the light path, adding an optical stretcher, or adding a variable fiber delay line using a fiber collimator and movable reflectors or fiber stretcher, or adding light guides or other transmissive features of higher or lower refractive index into the light path.

Adding Optical Switching

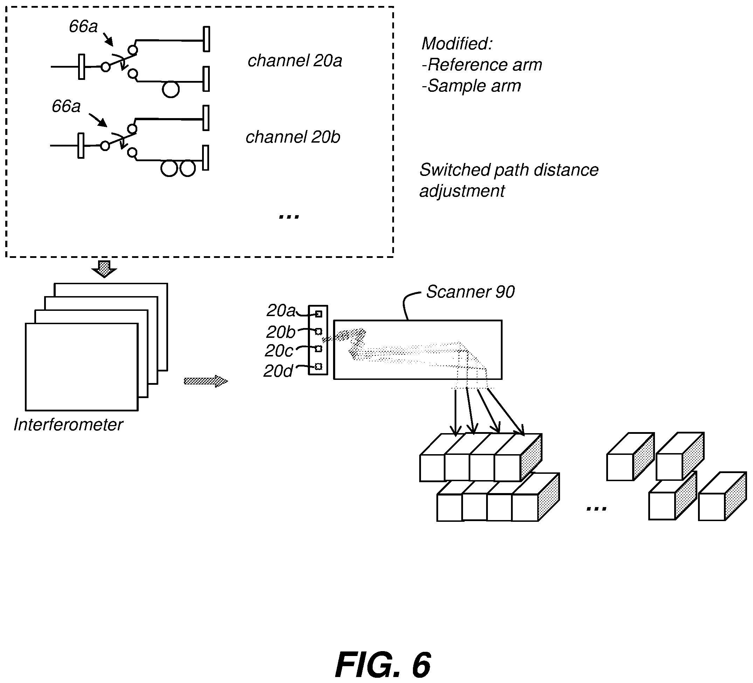

FIG. 6 displays a flexible way to extend the imaging range and to obtain various scanning patterns by adding an optical switch to each channel, wherein the optical switch selects alternate light paths of different optical path length. For the sake of example, two optical switches 66a and 66b for two channels 20a and 20b are shown; additional channels in an N-channel configuration could also be switched following the same pattern. It can also be noted that different switched patterns can be used to simultaneously scan different areas and different ranges using a swept-scan laser signal according to embodiments of the present disclosure. Thus, in the four-channel configuration schematically represented in FIG. 6, each channel can be switched to scan to a first range over its target sample region. The switching arrangement can then be changed to scan to a second range over the corresponding area of the sample. Multiple switch positions can be provided for each channel, allowing multiple optical path delays for any one or more channels and, as a result, multiple scan ranges. This sequence can achieve a large and adaptive imaging range with minimal motion artifacts.

It can readily be seen that using a switched delay arrangement with multiple scanning channels as represented in FIG. 6 allows the OCT scanning apparatus to extend and adapt imaging range without sacrificing scanning speed. Implementation of variable-range scanning can also be used to accommodate variables in surface contour, such as abrupt transitions in shape and contour characteristic of teeth and other intraoral features. A high-speed switcher can readily change the range settings between two or more scanning volumes, which provides the capability for real-time range adaptation.

ROI Scanning

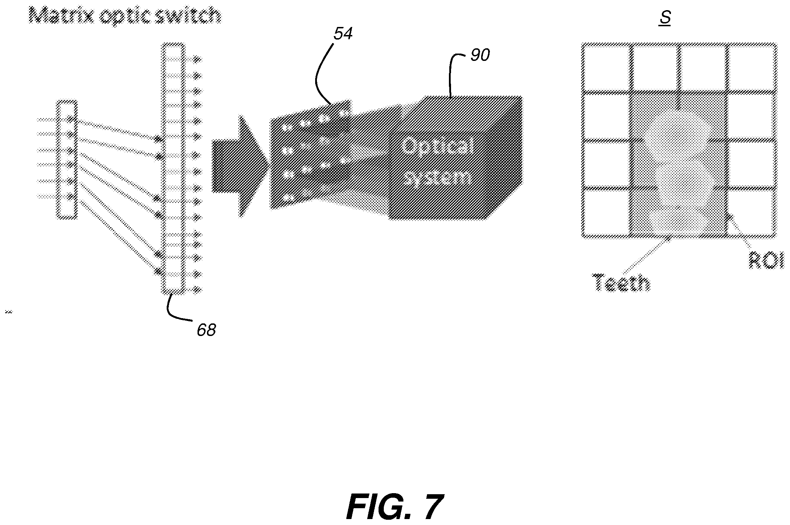

The multi-channel OCT system can also provide the option of adaptive region of interest (ROI) scanning. FIG. 7 illustrates a configuration for such ROI scanning, wherein a matrix optical switch 68 and a 2-D fiber array 54 are integrated with the scanner system. Using matrix switch 68 capabilities, incoming light from multiple channels is redistributed to multiple sub-regions in the FOV. The combined sub-regions define a region of interest (ROI) within the field of view. This configuration can effectively use the light to image a particular feature of interest at high speed. The capability to selectively shape the scanned region can dramatically reduce the volume of the data acquired for reconstruction and storage.

Additionally, by combining ROI selectivity with adjustable range scanning, as described previously with respect to FIGs. 5 and 6, example embodiments of the present invention can help to provide highly accurate OCT imaging results as the intraoral surface is scanned, without requiring significant computational resources and time.

Correction for Range Shifting

One inherent difficulty with multichannel embodiments relates to range shifting or z-axis offset between channels, due to factors related to OPD changes between the sample and reference arms. These shift offset effects can be due to variable factors related to cable routing and bending changes within the sample arm during handling, temperature shift, and vibration, for example, or mechanical drift of the optical mount. Relative range shifting, unless properly compensated, can introduce significant error in surface reconstruction. Although frequent calibration checks can help to compensate for static drift, the dynamic drift that results during handling and operation of the probe can be difficult to measure to sufficient levels of accuracy and without cumbersome instrumentation.

An example embodiment of the present invention compensates for the relative drift within each channel by employing an alternative back-scattering, reflective, or diffusive (i.e., diffused reflective) surface or feature that is disposed in a fixed position along the optical path as a spatial reference for measuring a corresponding range offset for the channel. The back-scattering, reflective, or diffusive feature can be formed in any of a number of ways, including formed by treatment of a surface that is part of the optical path or provided as a surface that is disposed at a fixed position in the optical path, such as at a predetermined, fixed position in the sample path, and within the field of view (FOV) of the intraoral scanner.

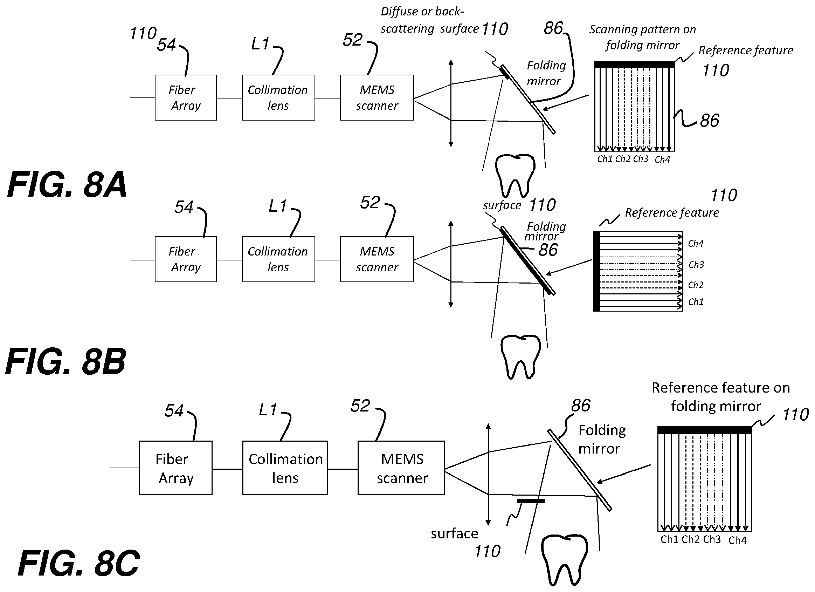

Referring to the schematic diagram of FIG. 8A, there is displayed the deployment of a diffusive or back-scattering reference surface or reference feature 110 as a range reference that is provided for scanning each OCT volume. Reference feature 110 is displayed in a number of alternative configurations in FIGs. 8A, 8B, and 8C. The diffusive or back-scattering surface of reference feature 110 can be a light scattering surface, such as a tape that adheres to the folding mirror 86 (FIGs. 8A, 8B) or is disposed in the path of scanned light, within the scanner FOV but spaced apart from mirror 86 (FIG. 8C). A pattern of features at known, predefined positions could alternately be used. The exact position of reference surface or feature 110 along the optical path is known and can be used as a range reference for adjusting/correcting the range of each acquired line of data.

With the configurations shown, each scan by a channel (during scan portion 92 of FIG. 2A) directs light to reference surface or feature 110. The returned light from diffusive or back-scattering reference feature 110 can be processed as part of the sample arm light within the interferometer system for the channel (FIG. 1). By scattering the bulk of the incident light it receives from the scanner 52 at the beginning or end of each scan line, or at known points in the scan line, reference feature 110 provides a strong signal indicative of the relative range of the scanned line data that corresponds to reference feature 110.

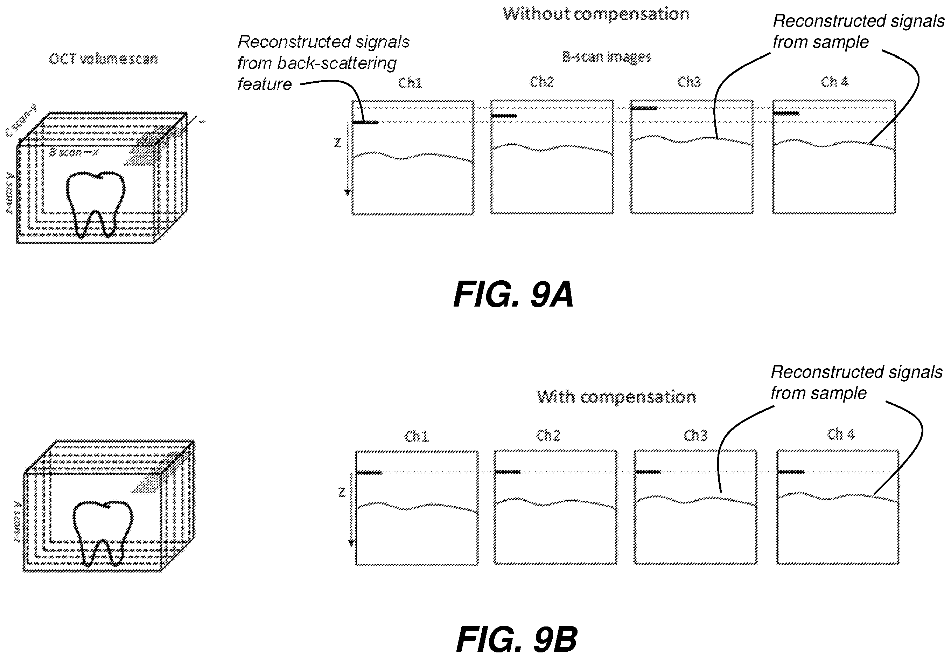

The schematic diagrams of FIGs. 9A and 9B show how variable range data for each channel can be compensated and normalized in order to provide OCT data that accurately represents the imaged surface. As FIG. 9A shows, the scanned data originally obtained has inherent range discrepancies between adjacent channels. By adjusting z-axis offset of the obtained data accordingly, as shown schematically in FIG. 9B, the differences in surface height can be correctly compensated.

The OCT signal from diffusive or back-scattering surface or other type of reference feature 110 can also be used to measure the intensity variation, or monitor the status of scanner and laser, such as to determine that the laser or scanner are active and operating, for example. Additionally, reference feature 110 can be used to resample the OCT signal and represent the OCT signal in a linear wavenumber space without using MZI 28, where the dispersion variation of the optical fiber during scanning can be eliminated.

A method for OCT scanning disposes a reference feature in the path of scanned light in the sample arm, wherein the reference feature redirects a portion of the scanned light back through the sample arm and to a detector for k-clock sampling and synchronization.

Using Polarization

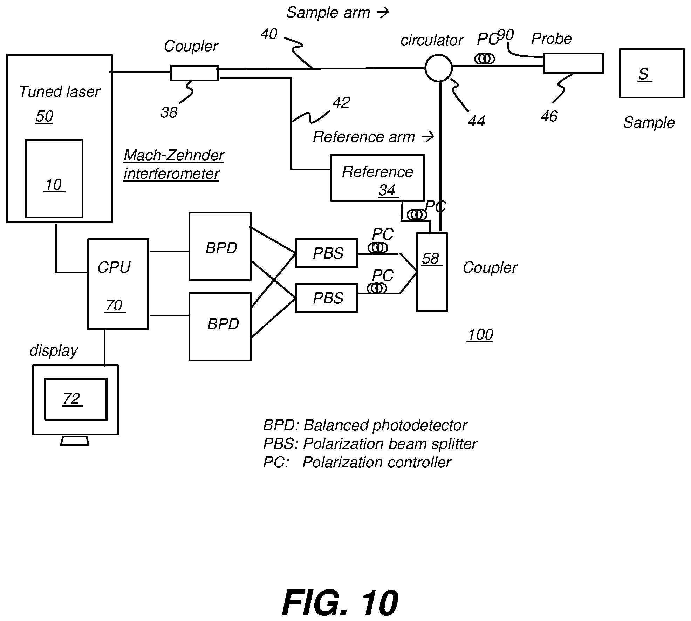

According to an alternate embodiment of the OCT imaging system, polarization selective OCT can be provided. This imaging method can be used to show aspects of materials interaction within the sample, for example. The schematic diagram of FIG. 10 displays a modification of the Mach-Zehnder interferometer with added polarization capability. Additional polarization controllers ("PC") can be provided on the sample and reference paths or arms to provide and process polarized light directed to the sample. One or more polarization beam splitter ("PBS") can direct the light of each polarization state to a suitable balanced photodetector ("BPD") input. The detected output can provide information related to the sample or other data that is available using polarized sample light, for example. The OCT system can optionally include additional detectors and optical components to provide polarization sensitive optical coherence tomography.

Artifact Suppression

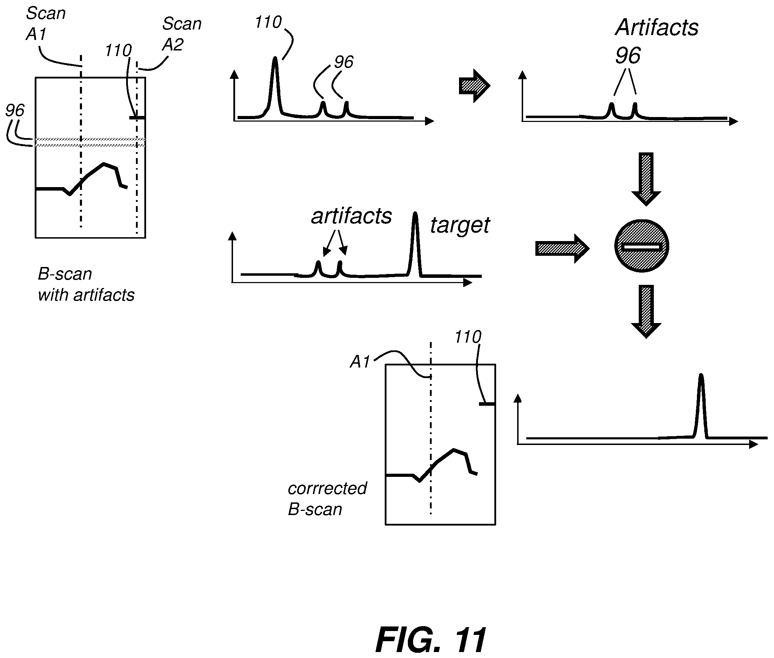

As displayed schematically in the sequence of FIG. 11, reference feature 110 can also be used for signal conditioning, such as artifact removal or suppression. Internal reflections within the optical system can generate horizontal line artifacts 96 in the B-scan image. Those artifacts may shift position within the image when the optical cable is twisted or bent. Under some conditions, artifacts 96 may even overlap with the actual signal from the sample S, making it difficult to distinguish between artifacts and the actual signal content.

A sequence to correct for this type of artifact and effectively remove it from the A-scan signal is as follows and is shown in FIG. 11:

(i) retrieve A-scan signals, including the reference feature and any artifacts;
(ii) set the amplitude of reference feature 110 as the background (or base noise) level; and
(iii) subtract the A-scan signal from other A-scans in the B-scan image.

In FIG. 11, scan A1 is a representative scan that does not include reference feature 110. Scan A2 is a scan that includes feature 110. The sequence effectively removes feature 110 content from scan A2 to isolate the artifact 96 content. The artifact 96 content can then be subtracted from any of the other scans A1 of the sample S. The final result is then free of the artifacts.

Example embodiments of the present invention show improvements for expanding the imaging range as well as increasing the effective speed of OCT scanning, both without requiring an increase in the scanner speed or improved digitizer response time. It should be appreciated and understood that various arrangements of the OCT scanner system can also achieve both increased speed and enhanced range, with corresponding changes to system design as taught herein.

The present invention has been described above in detail with particular reference to presently understood exemplary embodiments, but it should be appreciated and understood that variations and modifications may be affected within the scope of the appended claims. For example, control logic processor 70 can be any of a number of types of logic processing device, including a computer or computer workstation, a dedicated host processor, a microprocessor, logic array, or other device that executes stored program logic instructions. The interferometer that is used for one or more channels, described in the example configurations given hereinabove as a type of Mach-Zehnder interferometer, can alternatively be another appropriate type, such as a Michelson interferometer, for example, with appropriate component re-arrangement.

The presently disclosed exemplary embodiments are, therefore, considered in all respects to be illustrative and not restrictive. The scope of the present invention is indicated by the appended claims.

Consistent with at least one exemplary embodiment, exemplary methods/apparatus can use a computer program with stored instructions that perform on image data that is accessed from an electronic memory. As can be appreciated by those skilled in the image processing arts, a computer program of an exemplary embodiment herein can be utilized by a suitable, general-purpose computer system, such as a personal computer or workstation. However, many other types of computer systems can be used to execute the computer program of described example embodiments, including for example, an arrangement of one or networked processors.

A computer program for performing methods of certain example embodiments described herein may be stored in a computer readable storage medium. This medium may comprise, for example; magnetic storage media such as a magnetic disk such as a hard drive or removable device or magnetic tape; optical storage media such as an optical disc, optical tape, or machine readable optical encoding; solid state electronic storage devices such as random access memory (RAM), or read only memory (ROM); or any other physical device or medium employed to store a computer program. Computer programs for performing methods of described example embodiments may also be stored on computer readable storage medium that is connected to the image processor by way of the Internet or other network or communication medium. Those skilled in the art will further readily recognize that the equivalent of such a computer program product may also be constructed in hardware.

It should be noted that the term "memory", equivalent to "computer-accessible memory" in the context of the application, can refer to any type of temporary or more enduring data storage workspace used for storing and operating upon image data and accessible to a computer system, including for example, a database. The memory could be non-volatile, using, for example, a long-term storage medium such as magnetic or optical storage. Alternatively, the memory could be of a more volatile nature, using an electronic circuit, such as random-access memory (RAM) that is used as a temporary buffer or workspace by a microprocessor or other control logic processor device. Display data, for example, is typically stored in a temporary storage buffer that can be directly associated with a display device and is periodically refreshed as needed in order to provide displayed data. This temporary storage buffer can also be considered to be a memory, as the term is used in the application. Memory is also used as the data workspace for executing and storing intermediate and final results of calculations and other processing. Computer-accessible memory can be volatile, non-volatile, or a hybrid combination of volatile and non-volatile types.

It should be appreciated and understood that computer program products for example embodiments herein may make use of various image manipulation algorithms and/or processes that are well known. It should be further appreciated and understood that example computer program product embodiments herein may embody algorithms and/or processes not specifically shown or described herein that are useful for implementation. Such algorithms and processes may include conventional utilities that are within the ordinary skill of the image processing arts. Additional aspects of such algorithms and systems, and hardware and/or software for producing and otherwise processing the images or co-operating with the computer program product of the application, are not specifically shown or described herein and may be selected from such algorithms, systems, hardware, components and elements known in the art.

Example embodiments according to the present invention can include various features described herein (individually or in combination).

**Claims**

1. A dental optical coherence tomography system (150) for scanning a sample (S), the system (150) comprising:

   a) a swept source laser (50) configured to generate an output light having a range of light wavelengths;
   b) two or more optical channels (20, 20a, 20b, 20c, 20d, 20n),

      wherein each optical channel (20, 20a, 20b, 20c, 20d, 20n) provides a reference path (42) and a sample path (40) for the output light from the swept source laser (50),
      wherein each optical channel (20, 20a, 20b, 20c, 20d, 20n) has a corresponding detector (60) that is configured to provide an output signal according to combined light from the sample and reference paths (40, 42), and
      wherein the detector output signal characterizes back-reflected or back-scattered light returned from the sample path (40) and over a range of depths below a sample surface;

   c) a scanner (90) that is configured to simultaneously direct sample path output light from each of the two or more optical channels (20, 20a, 20b, 20c, 20d, 20n) toward the sample surface and to direct the returned light from the sample (S) to the corresponding sample path (40) and detector (60), the scanner (90) being incorporated into a handheld probe (46); and

   d) a processor (70) that is in signal communication with the detector (60) for each optical channel (20, 20a, 20b, 20c, 20d, 20n) and that is configured to record and store results from the output signals received from each detector (60).

2. The system (150) of claim 1, wherein the system (150) further comprises a camera (62) for detecting movement of the probe (46) or obtaining color information related to the sample (S).

3. The system (150) of claim 1, wherein the processor (70) is further configured to reconstruct a sample 2D section or 3D volume from the stored output signal results.

4. The system (150) of claim 1, wherein the scanner (90) comprises a MEMS reflector (52).

5. The system (150) of claim 1, wherein the system (150) further comprises an optical fiber array (54) that is configured to distribute the swept-source laser light to the two or more optical channels (20, 20a, 20b, 20c, 20d, 20n);
   wherein preferably the optical fiber array (54) is a 1-D or 2-D array.

6. The system (150) of claim 1, wherein the system (150) further comprises an optical switch (66a, 66b, 66n) configured to direct the output light:

   a) within an optical channel of the two or more optical channels (20, 20a, 20b, 20c, 20d, 20n), wherein a first position of the optical switch (66a, 66b, 66n) is configured to direct the output light over a first optical path length and the second position of the optical switch (66a, 66b, 66n) is configured to direct the output light over a second optical path length that is shorter than the first optical path length; or
   b) to a first or a second optical channel of the two or more optical channels (20, 20a, 20b, 20c, 20d, 20n).

7. The system (150) of claim 1, wherein the system (150) further comprises a back-scattering, reflective, or diffusive reference feature (110) disposed at a predetermined, fixed position in the sample path (40) and within a field of view of the scanner (90);

   wherein preferably detection of the reference feature (110) is used to compensate the optical path length difference between each optical channel (20, 20a, 20b, 20c, 20d, 20n);
wherein preferably detection of the reference feature (110) is used to monitor the intensity change of each optical channel (20, 20a, 20b, 20c, 20d, 20n) and compensate the intensity variation accordingly;
wherein preferably detection of the reference feature (110) is used to monitor the status of the laser (50) or the scanner (90);
wherein preferably detection of the reference feature (110) is used to remove artifacts from the returned light from the sample (S); or
wherein preferably a signal indicating detection of the reference feature (110) is used in resampling an OCT signal into a linear wavenumber space.

8. The system (150) of claim 1, wherein the system (150) further comprises one or more polarization beam splitters (32) disposed to provide polarization sensitive optical coherence tomography.

9. The system (150) of claim 1, wherein each reference path (42) is further configured as an adjustable optical delay line with a reflector or an optical stretcher.

10. The system (150) of claim 1, wherein the sample paths (40) comprise a plurality of optical fibers.

11. The system (150) of claim 1, wherein the sample paths (40) for the two or more optical channels (20, 20a, 20b, 20c, 20d, 20n) are spaced apart on the sample surface to form 1D or 2D arrays of scanned regions.

12. The system( 150) of claim 1, wherein corresponding optical path lengths in the reference and sample paths (40, 42) of the two or more channels (20, 20a, 20b, 20c, 20d, 20n) differ for scanning different imaging ranges.

13. A computer-implemented method for hand-held dental optical coherence tomography for imaging a sample using the system of any of claims 1-12, the method comprising the steps of:

   a) energizing a swept source laser to generate an output light having a range of light wavelengths;
   b) directing the output laser light through two or more optical channels, wherein each optical channel has a reference path and a sample path for the output light from the swept source laser, wherein each optical channel has a corresponding detector that is configured to provide an output signal according to combined light from the sample and reference paths, and wherein the detector output signal characterizes back-reflected or back-scattered light returned from the sample path and over a range of depths below a sample surface;
   c) configuring a scanning reflector to simultaneously direct sample path output light from each of the two or more optical channels toward the sample surface and to direct the returned light from the sample to the corresponding sample path and detector within the channel;
   d) for each optical channel, recording results from the output signals received from each detector; and
   e) reconstructing scanned portions of the sample according to the recorded results and displaying the reconstructed portions.

14. The method of claim 13, wherein the method further comprises a step of detecting a reflecting, absorbing, or back-scattering reference feature in the sample path and conditioning scan timing according to the detection.

15. The method of claim 13, wherein the method further comprises a step of detecting a reflecting, absorbing, or back-scattering reference feature in the sample path and suppressing one or more image artifacts according to the detection.

**Patentansprüche**

1. Optisches dentales Kohärenztomografiesystem (150) zum Abtasten einer Probe (S), wobei das System (150) umfasst:

   a) einen Swept-Source-Laser (50), der ausgebildet ist, ein einen Bereich von Lichtwellenlängen aufweisendes Ausgangslicht zu erzeugen;
   b) zwei oder mehr optische Kanäle (20, 20a, 20b, 20c, 20d, 20n),

      wobei jeder optische Kanal (20, 20a, 20b, 20c, 20d, 20n) einen Referenzpfad (42) und einen Probenpfad (40) für das Ausgangslicht von dem Swept-Source-Laser (50) bereitstellt,
      wobei jeder optische Kanal (20, 20a, 20b, 20c, 20d, 20n) einen entsprechenden Detektor (60) aufweist, der ausgebildet ist, ein Ausgangssignal gemäß kombiniertem Licht aus den Proben- und Referenzpfaden (40, 42) bereitzustellen, und
      wobei das Detektorausgangssignal rückreflektiertes oder rückgestreutes Licht, das von dem Probenpfad (40) zurückgegeben wird und aus einem Bereich von Tiefen unterhalb einer Probenoberfläche stammt, charakterisiert;

c) einen Scanner (90), der ausgebildet ist, Probenpfad-Ausgangslicht aus jedem der zwei oder mehr optischen Kanäle (20, 20a, 20b, 20c, 20d, 20n) gleichzeitig in Richtung auf die Probenoberfläche zu richten und das zurückgegebene Licht von der Probe (S) zu dem entsprechenden Probenpfad (40) und Detektor (60) zu richten, wobei der Scanner (90) in eine Handsonde (46) integriert ist; und

d) einen Prozessor (70), der in Signalverbindung mit dem Detektor (60) für jeden optischen Kanal (20, 20a, 20b, 20c, 20d, 20n) steht und der ausgebildet ist, Ergebnisse aus den von jedem Detektor (60) empfangenen Ausgangssignalen aufzuzeichnen und zu speichern.

2. System (150) nach Anspruch 1, wobei das System (150) weiter eine Kamera (62) umfasst, um eine Bewegung der Sonde (46) zu detektieren oder Farbinformationen zu erhalten, die sich auf die Probe (S) beziehen.

3. System (150) nach Anspruch 1, wobei der Prozessor (70) weiter ausgebildet ist, einen 2D-Abschnitt der Probe oder ein 3D-Volumen aus den gespeicherten Ausgangssignalergebnissen zu rekonstruieren.

4. System (150) nach Anspruch 1, wobei der Scanner (90) einen MEMS-Reflektor (52) umfasst.

5. System (150) nach Anspruch 1, wobei das System (150) weiter ein optisches Faserarray (54) umfasst, das ausgebildet ist, das Swept-Source-Laserlicht auf die zwei oder mehr optischen Kanäle (20, 20a, 20b, 20c, 20d, 20n) zu verteilen;

wobei bevorzugt das optische Faserarray (54) ein 1-D- oder 2-D-Array ist.

6. System (150) nach Anspruch 1, wobei das System (150) weiter einen optischen Schalter (66a, 66b, 66n) umfasst, der ausgebildet ist, das Ausgangslicht wie folgt zu leiten:

a) innerhalb eines optischen Kanals der zwei oder mehr optischen Kanäle (20, 20a, 20b, 20c, 20d, 20n), wobei eine erste Position des optischen Schalters (66a, 66b, 66n) ausgebildet ist, das Ausgangslicht über eine erste optische Weglänge zu leiten, und die zweite Position des optischen Schalters (66a, 66b, 66n) ausgebildet ist, das Ausgangslicht über eine zweite optische Weglänge zu leiten, die kürzer als die erste optische Weglänge ist; oder

b) zu einem ersten oder einem zweiten optischen Kanal der zwei oder mehr optischen Kanäle (20, 20a, 20b, 20c, 20d, 20n).

7. System (150) nach Anspruch 1, wobei das System (150) weiter ein rückstreuendes, reflektierendes oder diffuses Referenzmerkmal (110) umfasst, das an einer vorbestimmten, festen Position in dem Probenpfad (40) und innerhalb eines Sichtfelds des Scanners (90) angeordnet ist;

wobei bevorzugt die Detektion des Referenzmerkmals (110) verwendet wird, um die optische Weglängendifferenz zwischen jedem optischen Kanal (20, 20a, 20b, 20c, 20d, 20n) zu kompensieren;

wobei bevorzugt die Detektion des Referenzmerkmals (110) verwendet wird, um die Intensitätsänderung jedes optischen Kanals (20, 20a, 20b, 20c, 20d, 20n) zu überwachen und die Intensitätsschwankung entsprechend zu kompensieren;

wobei bevorzugt die Detektion des Referenzmerkmals (110) verwendet wird, um den Status des Lasers (50) oder des Scanners (90) zu überwachen;

wobei bevorzugt die Detektion des Referenzmerkmals (110) verwendet wird, um Artefakte aus dem von der Probe (S) zurückgegebenen Licht zu entfernen; oder

wobei bevorzugt ein Signal, das eine Detektion des Referenzmerkmals (110) anzeigt, bei der Neuabtastung eines OCT-Signals in einen linearen Wellenzahlraum verwendet wird.

8. System (150) nach Anspruch 1, wobei das System (150) weiter einen oder mehrere Polarisationsstrahlteiler (32) umfasst, die angeordnet sind, um polarisationssensitive optische Kohärenztomografie bereitzustellen.

9. System (150) nach Anspruch 1, wobei jeder Referenzpfad (42) weiter als eine einstellbare optische Verzögerungsleitung mit einem Reflektor oder einem optischen Strecker ausgebildet ist.

10. System (150) nach Anspruch 1, wobei die Probenpfade (40) eine Vielzahl von optischen Fasern umfassen.

11. System (150) nach Anspruch 1, wobei die Probenpfade (40) für die zwei oder mehr optischen Kanäle (20, 20a, 20b, 20c, 20d, 20n) auf der Probenoberfläche beabstandet sind, um 1D- oder 2D-Arrays von abgetasteten Regionen zu bilden.

12. System (150) nach Anspruch 1, wobei sich entsprechende optische Weglängen in den Referenz- und Probenpfaden (40, 42) der zwei oder mehr Kanäle (20, 20a, 20b, 20c, 20d, 20n) zum Abtasten unterschiedlicher Bildgebungsbereiche unterscheiden.

13. Computerimplementiertes Verfahren für handgehaltene dentale optische Kohärenztomografie zur Bildgebung einer Probe unter Verwendung des Systems nach einem der Ansprüche 1-12, wobei das Verfahren die folgenden Schritte umfasst:

a) Anregen eines Swept-Source-Lasers, um ein einen Bereich von Lichtwellenlängen aufweisendes Ausgangslicht zu erzeugen;

b) Leiten des Ausgangslaserlichts durch zwei oder mehr optische Kanäle, wobei jeder optische Kanal einen Referenzpfad und einen Probenpfad für das Ausgangslicht von dem Swept-Source-Laser aufweist, wobei jeder optische Kanal einen entsprechenden Detektor aufweist, der ausgebildet ist, ein Ausgangssignal gemäß kombiniertem Licht aus den Proben- und Referenzpfaden bereitzustellen, und wobei das Detektorausgangssignal rückreflektiertes oder rückgestreutes Licht, das von dem Probenpfad zurückgegeben wird und aus einem Bereich von Tiefen unterhalb einer Probenoberfläche stammt, charakterisiert;

c) Ausbilden eines Abtastreflektors, um Probenpfad-Ausgangslicht aus jedem der zwei oder mehr optischen Kanäle gleichzeitig in Richtung auf die Probenoberfläche zu richten und das zurückgegebene Licht von der Probe zu dem entsprechenden Probenpfad und Detektor innerhalb des Kanals zu richten;

d) für jeden optischen Kanal, Aufzeichnen von Ergebnissen aus den von jedem Detektor empfangenen Ausgangssignalen; und

e) Rekonstruieren abgetasteter Abschnitte der Probe nach den aufgezeichneten Ergebnissen und Anzeigen der rekonstruierten Abschnitte.

**14.** Verfahren nach Anspruch 13, wobei das Verfahren weiter einen Schritt des Detektierens eines reflektierenden, absorbierenden oder rückstreuenden Referenzmerkmals in dem Probenpfad und des Konditionierens des Scan-Timings entsprechend der Detektion umfasst.

**15.** Verfahren nach Anspruch 13, wobei das Verfahren weiter einen Schritt des Detektierens eines reflektierenden, absorbierenden oder rückstreuenden Referenzmerkmals in dem Probenpfad und des Unterdrückens eines oder mehrerer Bildartefakte entsprechend der Detektion umfasst.

**Revendications**

**1.** Système (150) de tomographie par cohérence optique dentaire pour balayer un échantillon (S), le système (150) comprenant :

a) un laser à source balayée (50) configuré pour générer une lumière de sortie présentant une plage de longueurs d'onde de lumière ;

b) deux ou plusieurs canaux optiques (20, 20a, 20b, 20c, 20d, 20n),

dans lequel chaque canal optique (20, 20a, 20b, 20c, 20d, 20n) fournit un trajet de référence (42) et un trajet d'échantillon (40) pour la lumière de sortie du laser à source balayée (50),

dans lequel chaque canal optique (20, 20a, 20b, 20c, 20d, 20n) présente un détecteur (60) correspondant qui est configuré pour fournir un signal de sortie selon une lumière combinée provenant des trajets d'échantillon et de référence (40, 42), et

dans lequel le signal de sortie du détecteur caractérise la lumière rétroréfléchie ou rétrodiffusée à partir du trajet d'échantillon (40) et sur une plage de profondeurs sous une surface d'échantillon ;

c) un réflecteur à balayage (90) qui est configuré pour diriger simultanément la lumière de sortie de trajet d'échantillon provenant de chacun des au moins deux canaux optiques (20, 20a, 20b, 20c, 20d, 20n) vers la surface d'échantillon et pour diriger la lumière renvoyée de l'échantillon (S) vers le trajet d'échantillon (40) et le détecteur (60) correspondants, le réflecteur à balayage (90) étant incorporé dans une sonde portative (46) ; et

d) un processeur (70) qui est en communication de signal avec le détecteur (60) pour chaque canal optique (20, 20a, 20b, 20c, 20d, 20n) et qui est configuré pour enregistrer et stocker des résultats à partir des signaux de sortie reçus de chaque détecteur (60).

**2.** Système (150) selon la revendication 1, dans lequel le système (150) comprend en outre une caméra (62) pour détecter un mouvement de la sonde (46) ou obtenir des informations de couleur relatives à l'échantillon (S).

**3.** Système (150) selon la revendication 1, dans lequel le processeur (70) est en outre configuré pour reconstruire une coupe 2D d'échantillon ou un volume 3D à partir des résultats de signaux de sortie stockés.

**4.** Système (150) selon la revendication 1, dans lequel le réflecteur à balayage (90) comprend un réflecteur MEMS (52).

**5.** Système (150) selon la revendication 1, dans lequel le système (150) comprend en outre un réseau de fibres optiques (54) qui est configuré pour répartir la lumière du laser à source balayée vers les au moins deux canaux optiques (20, 20a, 20b, 20c, 20d, 20n) ; dans lequel de préférence le réseau de fibres optiques (54) est un réseau 1-D ou 2-D.

**6.** Système (150) selon la revendication 1, dans lequel le système (150) comprend en outre un commutateur optique (66a, 66b, 66n) configuré pour diriger la lumière de sortie :

a) à l'intérieur d'un canal optique des au moins deux canaux optiques (20, 20a, 20b, 20c, 20d, 20n), dans lequel une première position du commutateur optique (66a, 66b, 66n) est configurée pour diriger la lumière de sortie sur une première longueur de trajet optique et la deuxième position du commutateur optique (66a, 66b, 66n) est configurée pour diriger la lumière de sortie sur une deuxième longueur de trajet optique qui est plus courte que la première longueur de trajet optique ; ou
b) vers un premier ou un deuxième canal optique des au moins deux canaux optiques (20, 20a, 20b, 20c, 20d, 20n).

**7.** Système (150) selon la revendication 1, dans lequel le système (150) comprend en outre un élément (110) de référence rétrodiffusant, réfléchissant, ou diffusant disposé à une position fixe prédéterminée dans le trajet d'échantillon (40) et dans un champ de vision du réflecteur à balayage (90) ;

dans lequel de préférence la détection de l'élément de référence (110) est utilisée pour compenser la différence de longueur de trajet optique entre chaque canal optique (20, 20a, 20b, 20c, 20d, 20n) ;
dans lequel de préférence la détection de l'élément de référence (110) est utilisée pour surveiller la variation d'intensité de chaque canal optique (20, 20a, 20b, 20c, 20d, 20n) et compenser en conséquence la variation d'intensité ;
dans lequel de préférence la détection de l'élément de référence (110) est utilisée pour surveiller l'état du laser (50) ou du réflecteur à balayage (90) ;
dans lequel de préférence la détection de l'élément de référence (110) est utilisée pour supprimer des artéfacts de la lumière retournée depuis l'échantillon (S) ; ou
dans lequel de préférence un signal indiquant la détection de l'élément de référence (110) est utilisé pour rééchantillonner un signal OCT dans un espace en nombre d'onde linéaire.

**8.** Système (150) selon la revendication 1, dans lequel le système (150) comprend en outre un ou plusieurs séparateurs de faisceau de polarisation (32) disposés pour fournir une tomographie par cohérence optique sensible à la polarisation.

**9.** Système (150) selon la revendication 1, dans lequel chaque trajet de référence (42) est en outre configuré comme une ligne à retard optique réglable avec un réflecteur ou un étireur optique.

**10.** Système (150) selon la revendication 1, dans lequel les trajets d'échantillon (40) comprennent une pluralité de fibres optiques.

**11.** Système (150) selon la revendication 1, dans lequel les trajets d'échantillon (40) pour les deux ou plusieurs canaux optiques (20, 20a, 20b, 20c, 20d, 20n) sont espacés les uns des autres sur la surface d'échantillon pour former des réseaux de régions balayées 1D ou 2D.

**12.** Système (150) selon la revendication 1, dans lequel des longueurs de trajet optique correspondantes dans les trajets de référence et d'échantillon (40, 42) des deux ou plusieurs canaux (20, 20a, 20b, 20c, 20d, 20n) diffèrent pour balayer différentes plages d'imagerie.

**13.** Procédé mis en œuvre par ordinateur pour la tomographie par cohérence optique dentaire portative pour imager un échantillon en utilisant le système selon l'une quelconque des revendications 1-12, le procédé comprenant les étapes consistant à :

a) mettre sous tension un laser à source balayée pour générer une lumière de sortie présentant une plage de longueurs d'onde de lumière ;
b) diriger la lumière de sortie du laser à travers deux ou plusieurs canaux optiques, dans lesquels chaque canal optique présente un trajet de référence et un trajet d'échantillon pour la lumière de sortie du laser à source balayée, dans lequel chaque canal optique présente un détecteur correspondant qui est configuré pour fournir un signal de sortie selon une lumière combinée provenant du trajet d'échantillon et du trajet de référence, et dans lequel le signal de sortie du détecteur caractérise la lumière rétroréfléchie ou rétrodiffusée retournée depuis le trajet d'échantillon et sur une plage de profondeurs sous une surface d'échantillon ;
c) configurer un réflecteur à balayage pour diriger simultanément la lumière de sortie du trajet d'échantillon provenant de chacun des au moins deux canaux optiques vers la surface d'échantillon et pour diriger la lumière retournée depuis l'échantillon vers le trajet d'échantillon et le détecteur correspondants dans le canal ;
d) pour chaque canal optique, enregistrer les résultats à partir des signaux de sortie reçus de chaque détecteur ; et

e) reconstruire des portions balayées de l'échantillon selon les résultats enregistrés et afficher les portions reconstruites.

14. Procédé selon la revendication 13, dans lequel le procédé comprend en outre une étape de détection d'un élément de référence réfléchissant, absorbant, ou rétrodiffusant dans le trajet d'échantillon et conditionnant la temporisation du balayage en fonction de la détection.

15. Procédé selon la revendication 13, dans lequel le procédé comprend en outre une étape de détection d'un élément de référence réfléchissant, absorbant ou rétrodiffusant dans le trajet d'échantillon et supprimant un ou plusieurs artéfacts d'image en fonction de la détection.

# FIG. 1

(Prior Art)

EP 3 937 758 B1

Full set of A-scan sweeps and acquisitions during each B-scan

Retro-scan 93

92

One B scan

Signal to scanner

Time

A-scan data

Each A-scan

Time

Acquired interference spectrum

88

Amplitude

FFT

Fourier transform of one A-scan

T

C scan-- y

93

82

Retro-scan

B scan-- x

Portion from one A-scan

B-scan image

**FIG. 2A**

(Prior Art)

EP 3 937 758 B1

Retro-
scan 93    82

C scan--y

Each row is a
B-scan

B scan--- x

C scan-y

B scan—x    z

A scan-z

Sequence of
B-scan images

# FIG. 2B

(Prior Art)

**FIG. 3A**

**FIG. 3B**

FIG. 3C

**FIG. 4A**

EP 3 937 758 B1

EP 3 937 758 B1

**FIG. 4B**

FIG. 5

EP 3 937 758 B1

FIG. 6

**FIG. 7**

EP 3 937 758 B1

Diffuse or back-
scattering surface
110 54   L1   52   Scanning pattern on
folding mirror
Reference feature

86   Folding
mirror
110

Fiber
Array   Collimation
lens   MEMS
scanner

Ch1 Ch2 Ch3 Ch4

**FIG. 8A**

54   L1   52   surface 110   110

Folding
mirror
86

Reference feature

Fiber
Array   Collimation
lens   MEMS
scanner

Ch4
Ch3
Ch2
Ch1

**FIG. 8B**

Reference feature on
folding mirror

54   L1   52   86   Folding
mirror   110

Fiber
Array   Collimation
lens   MEMS
scanner

surface
110

Ch1 Ch2 Ch3 Ch4

**FIG. 8C**

EP 3 937 758 B1

OCT volume scan

Without compensation

Reconstructed signals from back-scattering feature

B-scan images

Reconstructed signals from sample

Ch1     Ch2     Ch3     Ch 4

z

## FIG. 9A

With compensation

Reconstructed signals from sample

Ch1     Ch2     Ch3     Ch 4

z

## FIG. 9B

FIG. 10

FIG. 11

EP 3 937 758 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2011109911 A1